# EUROPEAN PATENT APPLICATION

(11) **EP 2 692 358 A1**
(43) Date of publication of application: **05.02.2014**
(21) Application number: 12765858.1
(22) Date of filing: 30.03.2012
(51) Int. Cl.: A61K 39/395, A61P 1/00, A61P 35/00, G01N 33/48, G01N 33/53, G01N 33/574, C07K 16/28

(54) **THERAPEUTIC AGENT FOR CANCER, AND METHOD FOR DETERMINING PROGNOSIS OF CANCER**

(30) Priority: 31.03.2011 JP 2011080261
(71) Applicant: Kyoto University, Kyoto-shi, Kyoto 606-8501 (JP)
(72) Inventor: TSUCHIYA, Soken, Kyoto-shi Kyoto 606-8501 (JP); TSUJIMOTO, Gozoh, Kyoto-shi Kyoto 606-8501 (JP); SHIMIZU, Kazuharu, Kyoto-shi Kyoto 606-8501 (JP); SHIMADA, Yutaka, Toyama-shi Toyama 930-0194 (JP); TSUKADA, Kazuhiro, Toyama-shi Toyama 930-0194 (JP)
(74) Representative: Keirstead, Tanis Evelyne
(86) International application number: PCT/JP2012/058668
(87) International publication number: WO 2012/133814

(57) **Abstract**

Disclosed are a novel therapeutic agent for cancer such as esophageal squamous cell carcinoma, a method for predicting the prognosis of cancer, and a method for detecting, or predicting the prognosis of, cancer such as esophageal squamous cell carcinoma using a sample that can be collected less invasively. The therapeutic agent for cancer comprises as an effective component an antibody that undergoes antigen-antibody reaction with FGFRL1 to suppress the growth of cancer cells, or an antigen-binding fragment thereof. The method for predicting the prognosis of cancer comprises investigating the expression level of FGFRL1 in a cancer tissue separated from a living body, and, in this method, a high expression level of FGFRL1 indicates poor prognosis. The method for detecting cancer comprises measuring FGFRL1 or a fragment thereof extracted from a body tissue, or FGFRL1 or a fragment thereof in blood separated from a living body, and, in this method, a higher concentration of FGFRL1 or the fragment thereof contained therein than the concentration of FGFRL1 or the fragment thereof in the tissue or blood of a healthy individual indicates the presence of cancer.

## Description

### TECHNICAL FIELD

The present invention relates to a therapeutic agent for cancer, a method for predicting the prognosis of cancer, and a method for detecting cancer.

### BACKGROUND ART

Esophagus cancer is a cancer with the eighth highest incidence rate and the sixth highest number of deaths. In Japan, esophageal squamous cell carcinoma (ESCC) accounts for not less than 90% of esophagus cancer. ESCC is a highly malignant cancer that frequently causes distant metastasis and recurrence, and its prognosis is generally poor.

On the other hand, it has been reported that ovarian cancer shows abnormal expression of fibroblast growth factor receptor like-1 (hereinafter referred to as "FGFRL1") (Non-patent Document 1). However, in this report, no statistical analysis was carried out for the expression level of FGFRL1, and no analysis on the function of FGFRL1 was performed. Thus, this report never leads to inference of promotion of the cell growth by FGFRL1, utilization of its expression level for prediction of the prognosis, or its industrial applicability. Further, although it has been reported that microRNA (miRNA)-210 is involved in oncogenesis and that one of its target genes is FGFRL1 (Non-patent Document 2), this report does not clearly suggest utilization of FGFRL1 for prediction of the prognosis or for therapeutic agents. Further, the present inventors previously discovered that, in esophageal squamous cell carcinoma, microRNA-210 regulates the growth of cancer cells via FGFRL1 (Non-patent Document 3). However, this report only elucidated that a target gene of microRNA-210 is FGFRL1 and discussed about its downstream pathway, and no suggestion was made about possible use of an anti-FGFRL1 antibody for a therapeutic agent for cancer or a tool for prediction of the prognosis.

On the other hand, although FGFRL1 has been named a "molecule like a fibroblast growth factor receptor (FGFR)", it is clearly structurally different from other FGFRs since, unlike other FGFRs, FGFRL1 lacks the tyrosine kinase domain, which is a signaling region in the cell (Non-patent Document 4). Further, the functional aspect of FGFRL1 is also different from other FGFRs, and FGFRL1 has been considered to act to suppress the cell growth (Non-patent Document 5). Accordingly, FGFRL1 is a molecule clearly distinguishable from other FGFRs, and should be clearly distinguished from the FGFRs also in view of its biological role and the industrial applicability deduced therefrom.

[Non-patent Document 1] International Journal of Molecular Medicine 16, 1169-1173,2005
[Non-patent Document 2] Molecular Cell 35, 856-867, 2009.
[Non-patent Document 3] Journal of Biological Chemistry 286, 420-428, 2011
[Non-patent Document 4] Genomics 69, 275-279, 2000
[Non-patent Document 5] Journal of Biological Chemistry 278, 33857-33865, 2003

### SUMMARY OF THE INVENTION

### PROBLEMS TO BE SOLVED BY THE INVENTION

An object of the present invention is to provide a novel therapeutic agent for cancer such as esophageal squamous cell carcinoma. Another object of the present invention is to provide a method for predicting the prognosis of cancer such as esophageal squamous cell carcinoma. Still another object of the present invention is to provide a method for detecting, or predicting the prognosis of, cancer such as esophageal squamous cell carcinoma using a sample that can be collected less invasively.

As described specifically in the Examples below, the present inventors collected esophageal squamous cell carcinoma tissues from a large number of esophageal squamous cell carcinoma patients to investigate expression of FGFRL1, and conducted a follow-up study on association of the expression level with the prognosis. As a result, it was found that high expression of FGFRL1 is associated with poor prognosis. Further, the present inventors discovered that allowing an anti-FGFRL1 antibody to act on esophageal squamous cell carcinoma cells enables suppression of the growth of the cancer cells.

That is, the present invention provides a therapeutic agent for cancer, comprising as an effective component an antibody that undergoes antigen-antibody reaction with FGFRL1 to suppress the growth of cancer cells, or an antigen-binding fragment thereof. Further, the present invention provides a therapeutic method for cancer, comprising administering to a cancer patient an effective amount of an antibody that undergoes antigen-antibody reaction with FGFRL1 to suppress the growth of cancer cells, or an antigen-binding fragment thereof. Further, the present invention provides a method for predicting the prognosis of cancer, comprising investigating the expression level of FGFRL1 in a cancer tissue separated from a living body, wherein a high expression level of FGFRL1 indicates poor prognosis. Further, the present invention provides a method for detecting cancer, comprising measuring FGFRL1 or a fragment thereof extracted from a body tissue, or FGFRL1 or a fragment thereof in blood separated from a living body, wherein a higher concentration of FGFRL1 or the fragment thereof contained therein than the concentration of FGFRL1 or the fragment thereof in the tissue or blood of a healthy individual indicates the presence of cancer. Further, the present invention provides a method for predicting the prognosis of cancer, comprising measuring FGFRL1 or a fragment thereof in a tissue or blood separated from a cancer patient, wherein a high concentration of FGFRL1 or the fragment thereof contained therein indicates poor prognosis.

### EFFECT OF THE INVENTION

By the present invention, a novel therapeutic agent for cancer such as esophageal squamous cell carcinoma was provided. Further, by the present invention, a novel method for predicting the prognosis of cancer such as esophageal squamous cell carcinoma was provided. Since this method enables prediction of the prognosis of a highly malignant cancer such as esophageal squamous cell carcinoma, and hence enables appropriate selection of a therapeutic method, the method contributes to treatment of cancer. Further, by the present invention, a method for detecting a cancer such as esophageal squamous cell carcinoma using a sample that can be collected less invasively was provided. Since this method is less invasive, the burden of the subject is light. Therefore, detection of cancer can be easily achieved also in medical examination and the like, and early detection and early treatment of cancer are possible.

### BRIEF DESCRIPTION OF THE DRAWINGS

Fig. 1 shows photographs for comparison of the results of immunohistochemistry of esophageal squamous cell carcinoma carried out in the Examples below with the results obtained for normal tissues.
Fig. 2 is a diagram showing the relationship between the expression level of FGFRL1 in the esophageal squamous cell carcinoma tissue measured in the Examples below and the survival rate of patients at each month.
Fig. 3 is a diagram showing the results of measurement of the growth capacity of esophageal squamous cell carcinoma cells observed after allowing an anti-FGFRL1 antibody to act on the cells in the Examples below, as compared to the results obtained by treatment with a control antibody.

### BEST MODE FOR CARRYING OUT THE INVENTION

As described above, the therapeutic agent for cancer of the present invention comprises as an effective component an antibody that undergoes antigen-antibody reaction with FGFRL1 or an antigen-binding fragment thereof. Both the amino acid sequence and the gene base sequence are known for FGFRL1. A base sequence of cDNA of FGFRL1 and the amino acid sequence encoded thereby are described, for example, as NCBI Accession NO. NM_001004356.2. The base sequence of cDNA of the FGFRL1 gene and the amino acid sequence encoded thereby are shown in SEQ ID NO:1, and the amino acid sequence alone is shown in SEQ ID NO:2. FGFRL1 is a single-transmembrane protein.

The antibody used herein is an antibody that suppresses the growth of cancer cells, and the antibody undergoes antigen-antibody reaction with the N-terminal region of FGFRL1, that is, the epitope of the antibody is preferably present in the N-terminal region of FGFRL1 or in a region comprising the whole or a part of the N-terminal region (that is, a region that extends from the N-terminal region to another region). The N-terminal region herein means the extracellular region of FGFRL1, that is, the region between the N-terminus and the 378th amino acid in the amino acid sequence shown in SEQ ID NO:2. The antibody may be either a polyclonal antibody or a monoclonal antibody. A polyclonal antibody whose epitope is the N-terminal region of FGFRL1 is also commercially available, and such a commercially available product may also be preferably used. Further, an antigen-binding fragment of the above-described antibody, such as the Fab fragment or the F(ab')₂ fragment, may also be used. Whether or not the antibody or fragment has an effect to suppress the growth of cancer cells can be investigated by, for example, using the well-known WST1 method as described in the Examples below.

The antibody may be an antibody prepared by genetic engineering, or may be a humanized antibody prepared by replacing the Fc region with that of a human antibody for suppression of rejection reaction in human. Further, in antibody preparations, those prepared by binding a polyethylene glycol (PEG) chain or the like to an end of an antibody for making the antibody less likely to be degraded by protease in the living body are widely used. Also in the therapeutic agent for cancer of the present invention, a stabilizing structure such as a PEG chain may be attached to an end of the above-described antibody or the antigen-binding fragment thereof, and the resultant may be contained in its entirety in the agent as an effective component. In cases where the antibody or an antigen-binding fragment thereof is stabilized by PEGylation, the size of the PEG is several thousand to 50,000, preferably about 10,000 to 50,000. The method for binding PEG to an end of a polypeptide is well known. Such a product prepared by attaching a stabilizing structure is also included in the "antibody or an antigen-binding fragment thereof' in the present invention.

Further, the present antibody may also be utilized as a complex antitumor agent by chemically binding a low molecular weight antitumor agent or a compound having cytotoxicity against cancer thereto, or may be utilized as a navigator in a drug delivery system (DDS) to cancer cells

In terms of the administration route of the therapeutic agent for cancer of the present invention, either parenteral administration or oral administration may be carried out. Parenteral administration such as injection to the cancer tissue, intravenous injection, or intramuscular injection is preferred. The dose may be appropriately set depending on the clinical condition and the severity of the disease to be treated. For example, the therapeutic agent is administered at a dose of 0.1 to 20 mg per administration, preferably 1 to 10 mg per administration, per kg body weight. Further, the therapeutic agent for cancer of the present invention may be formulated by a well-known method into, for example, a solution in which the agent is dissolved in a physiological buffer. Further, a known additive(s) may be added to the solution.

Examples of the cancer to be treated with the therapeutic agent for cancer of the present invention include, but are not limited to, epithelial solid cancers. Esophageal squamous cell carcinoma is especially preferred.

The present inventors discovered, as specifically described in the Examples below, that the expression level of FGFRL1 in a cancer tissue can be used as an index for predicting the prognosis of the cancer, that is, the survival rate after initiation of cancer treatment. Thus, the present invention also provides a method for predicting the prognosis of cancer, which method comprises investigating the expression level of FGFRL1 in a cancer tissue separated from a living body, wherein a high expression level of FGFRL1 indicates poor prognosis. The expression level of FGFRL1 can be measured by an immunoassay such as immunohistochemistry. For the immunoassay, the above-described anti-FGFRL1 antibody or an antigen-binding fragment thereof may be used, and a polyclonal antibody or monoclonal antibody whose epitope is the extracellular region of FGFRL1 may be preferably used. Since, as described above, such a polyclonal antibody is also commercially available, it is also possible to use a commercially available product. Since FGFRL1 is expressed in a state where it is penetrating the membrane, the immunohistochemical staining as described in the Examples below is preferably carried out as the immunoassay, but the immunoassay is not limited thereto.

The higher the expression level of FGFRL1, the poorer the prognosis may be. Thus, by preliminarily investigating the expression level of FGFRL1 and the prognosis in a large number of patients with the same kind of cancer, it is possible to predict the prognosis based on how high the expression level of FGFRL1 is. For example, as specifically described in the Examples below, in cases where the expression level is investigated by immunohistochemical staining, the prediction may be made based on evaluation of the stained area per cancer tissue (0%: -, 1-50%: +, 51-100%: ++), and the positivity per cell wherein strong positivity is evaluated as (+++) and negativity is evaluated as (-). In consideration of the extent of expression of FGFRL1 in a normal tissue, a total value of not less than (++) can be judged as positive (that is, poor prognosis).

As described above, FGFRL1 is a single-transmembrane protein, and has a structure that undergoes the action of protease in the extracellular region. Therefore, it is thought that a tissue fluid extracted from a tissue, or blood, may contain free FGFRL1 or a free fragment of FGFRL1. The present inventors inferred that, by quantifying free FGFRL1 or a free fragment of FGFRL1 in a tissue fluid extracted from a tissue, or blood, cancer can be detected. The present invention also provides a method for detecting cancer, which method comprises measuring FGFRL1 or a fragment thereof extracted from a body tissue, or FGFRL1 or a fragment thereof in blood separated from a living body, wherein a higher concentration of FGFRL1 or the fragment thereof contained therein than the concentration of FGFRL1 or the fragment thereof in the tissue or blood of a healthy individual indicates the presence of cancer. In such cases, the FGFRL1 or a fragment thereof in the tissue extract or blood can be quantified by an immunoassay using an antibody that undergoes antigen-antibody reaction with the extracellular region of FGFRL1. The immunoassay in such cases may be carried out by a well-known method such as ELISA, which is widely used for quantification of various proteins in body fluid; the sandwich method, in which a fluorescent label or chemiluminescent label is used; or the immunoagglutination method, in which sensitized particles prepared by immobilizing an antibody on latex particles are used. The cut-off value in such cases may be a value significantly different from the mean value in healthy individuals. The cut-off value may be, for example, 1.0 unit/mL, and the unit value in such cases is determined using as a standard the concentration in the tissue or blood of a healthy individual, although the unit value may vary depending on differential diagnosis from similar diseases and on background factors of the patient.

Further, based on the abundance of FGFRL1 or a fragment thereof in a tissue fluid extracted from a tissue, or blood, prediction of the prognosis of cancer can be carried out similarly to the above-described cases where the prediction is carried out based on the expression level of FGFRL1 in the cancer tissue. In such a case, the criteria for evaluation of the prognosis may vary depending on whether the survival rate or the recurrence rate is to be evaluated, and for what disease the evaluation is to be done.

The present invention is described below more specifically by way of Examples. However, the present invention is not limited to the Examples below.

### EXAMPLES

### Example 1

### Immunohistochemical Staining

Tissue sections were prepared from esophageal squamous cell carcinoma tissues collected from 69 esophageal squamous cell carcinoma patients, and subjected to deparaffinization (3 times of 5 minutes of immersion in xylene, 2 times of 3 minutes of immersion in 100% ethanol, 3 minutes of immersion in 95% ethanol, 3 minutes of immersion in 90% ethanol, 3 minutes of immersion in 85% ethanol, 5 minutes of washing with running water, and then 5 minutes of immersion in distilled water) and then antigen retrieval by heat treatment (treatment in 1 mM Tris buffer pH 9.0) supplemented with 0.1 mM EDTA at 95°C for 40 minutes, followed by allowing the resultant to cool at room temperature for 20 minutes, washing with running water and then immersion in distilled water). Subsequently, endogenous peroxidase was blocked with 3% hydrogen peroxide solution (at room temperature for 10 minutes), and the sections were then washed with distilled water 3 times, followed by immersion in 5 mM Tris buffer (pH 7.2) supplemented with 0.005% Tween 20 and 15 mM NaCl at room temperature for 5 minutes for achieving equilibration. Anti-FGFRL1 antibody H-300 (Santa cruse) was 50-fold diluted with Dako REAL antibody diluent (Dako), and treatment was carried out with the resulting dilution for 30 minutes. After washing the sections with 5 mM Tris buffer (pH 7.2) supplemented with 0.005% Tween 20 (trade name) and 15 mM NaCl 3 times, coloring with DAB (diaminobenzidine) was performed using Dako ChemMate ENVISION kit (Dako), followed by washing with distilled water and then performing counter staining with Dako REAL Hematoxylin (prepared by 4-fold dilution with distilled water and then addition of Tween 20 (trade name) to adjust the Tween 20 concentration to 0.01 %) at room temperature for 3 minutes. Thereafter, washing with water, dehydration, clearing and embedding were carried out (5 minutes of washing with water, 5 minutes of immersion in 80% ethanol, 5 minutes of immersion in 90% ethanol, 5 minutes of immersion in 95% ethanol, 2 times of 5 minutes of immersion in 100% ethanol, and 3 times of 5 minutes of immersion in xylene, followed by embedding with Leica CV5030). The stained area per cancer tissue (0%: 0, 1-50%: 1, 51-100%: 2) and the staining intensity (no signal: 0, weak: 1, moderate: 2, marked: 3) were scored, and a total score of not less than 4 was defined as high expression of the FGFRL1 protein, and a total score of less than 4 was defined as low expression of the FGFRL1 protein. The survival rate was compared between both groups of patients by the Kaplan-Meier method and the log-rank test.

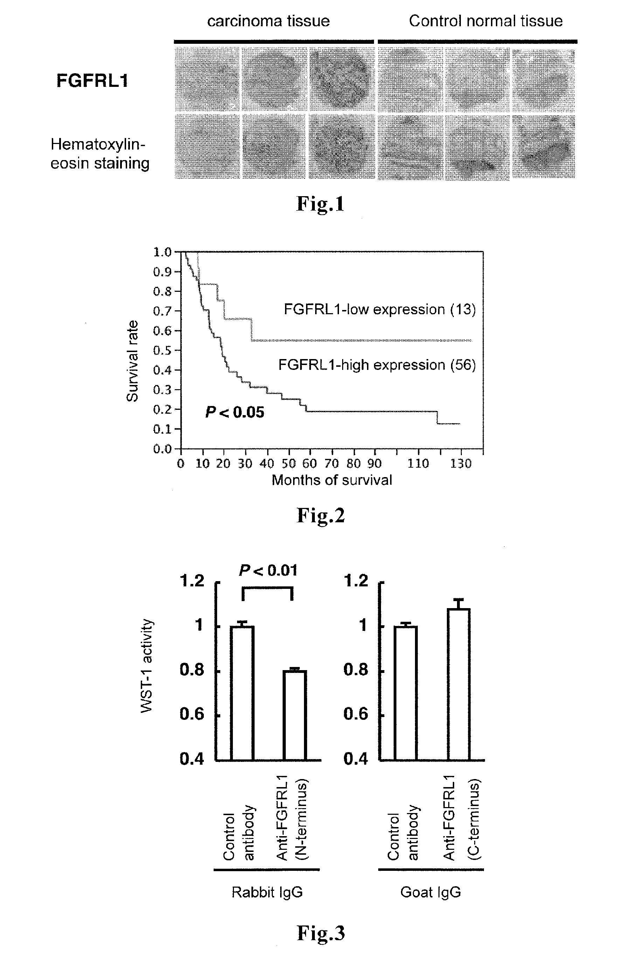

The results of immunohistochemical staining are shown in Fig. 1, and the relationship between the expression level of FGFRL1 and the survival rate of patients at each month is shown in Fig. 2. As is evident from Fig. 2, the prognosis was poor in the cases where the expression level of FGFRL1 was high, and the survival rate at Month 60 in these cases was a little more than one third of the survival rate observed in the cases where the expression level of FGFRL1 was low.

### Example 2

### Pharmacological Effect

An esophageal squamous cell carcinoma-derived cell line KYSE-170 was plated in Ham F12 (Nissui)/RPMI1640 (Gibco) medium (pH 6.8) supplemented with fetal bovine serum (5%, Equitech-Bio) filtered through a 0.22-m PVDF membrane filter (Millipore), penicillin (100 unit/ml, Meiji), gentacin (4.44 µmg/l, Schering) and sodium hydrogen carbonate (0.2%) on a 96-well dish (5×10³ cells/100 µL/well), and cultured under the conditions of 5% CO₂, a humidity of 100% and a temperature of 37°C. Twenty four hours later, the cells were treated with anti-FGFRL1 antibody H-300 (recognition site: N-terminus/extracellular region) or C-20 (recognition site: C-terminus/intracellular region) (Santa cruse) and a control IgG (Santa cruse) of the animal from which it was derived, which were diluted with the above-described Ham F12/RPMI1640 medium (final concentration, 20 µg/ml). After 24 hours of culture, the cell growth was evaluated by the well-known WST1 method using a commercially available reagent.

The results are shown in Fig. 3. As shown in Fig. 3, in the case where the monoclonal antibody whose epitope is the N-terminal region, that is, the extracellular region, of FGFRL1 was used, the growth of esophageal squamous cell carcinoma cells was significantly suppressed as compared to the case where the treatment was carried out with the control antibody. Thus, such an antibody is useful as a therapeutic agent for esophageal squamous cell carcinoma.

## Claims

1. A therapeutic agent for cancer, said therapeutic agent comprising as an effective component an antibody that undergoes antigen-antibody reaction with FGFRL1 to suppress growth of cancer cells, or an antigen-binding fragment thereof.

2. The therapeutic agent for cancer according to claim 1, wherein said antibody is an antibody that undergoes antigen-antibody reaction with the N-terminal region of FGFRL1.

3. The therapeutic agent for cancer according to claim 1 or 2, wherein said cancer is esophageal squamous cell carcinoma.

4. A therapeutic method for cancer, said therapeutic method comprising administering to a cancer patient an effective amount of an antibody that undergoes antigen-antibody reaction with FGFRL1 to suppress growth of cancer cells, or an antigen-binding fragment thereof.

5. The method according to claim 4, wherein said antibody is an antibody that undergoes antigen-antibody reaction with the N-terminal region of FGFRL1.

6. The method according to claim 4 or 5, wherein said cancer is esophageal squamous cell carcinoma.

7. A method for predicting the prognosis of cancer, said method comprising investigating the expression level of FGFRL1 in a cancer tissue separated from a living body, wherein a high expression level of FGFRL1 indicates poor prognosis.

8. The method according to claim 7, wherein said cancer is esophageal squamous cell carcinoma.

9. The method according to claim 7 or 8, wherein the expression level of FGFRL1 is investigated by immunohistochemistry

10. A method for detecting cancer, said method comprising measuring FGFRL1 or a fragment thereof extracted from a body tissue, or FGFRL1 or a fragment thereof in blood separated from a living body, wherein a higher concentration of FGFRL1 or the fragment thereof contained therein than the concentration of FGFRL1 or the fragment thereof in the tissue or blood of a healthy individual indicates the presence of cancer.

11. The method according to claim 10, wherein said cancer is esophageal squamous cell carcinoma.

12. A method for predicting the prognosis of cancer, said method comprising measuring FGFRL1 or a fragment thereof in a tissue or blood separated from a cancer patient, wherein a high concentration of FGFRL1 or the fragment thereof contained therein indicates poor prognosis.

13. The method according to claim 12, wherein said cancer is esophageal squamous cell carcinoma.


### [SEQUENCE LISTING]

SEQUENCE LISTING

<110>  Kyoto University

<120>  Therapeutic agent of cancers and method of predicting prognosis of
cancers

<130>  PF454-PCT

<160>  2

<170>  PatentIn version 3.1

<210>  1
<211>  3215
<212>  DNA
<213>  Homo sapiens

<220>
<221>  CDS
<222>  (150)..(1664)
<223>

<400>  1
gccgccccgc cccgagaccg ggcccggggg cgcggggcgg cgggatgcgg cgcccggggc      60

ggcgatgacc gcggagcgca cgccgcgggc ccggccctga ccccgccgcc cgcccgctga     120

gcccccgcc gaggtccgga caggccgag atg acg ccg agc ccc ctg ttg ctg      173
                              Met Thr Pro Ser Pro Leu Leu Leu
                              1               5

ctc ctg ctg ccg ccg ctg ctg ctg ggg gcc ttc ccg ccg gcc gcc gcc      221
Leu Leu Leu Pro Pro Leu Leu Leu Gly Ala Phe Pro Pro Ala Ala Ala
    10                  15                  20

gcc cga ggc ccc cca aag atg gcg gac aag gtg gtc cca cgg cag gtg      269
Ala Arg Gly Pro Pro Lys Met Ala Asp Lys Val Val Pro Arg Gln Val
25                  30                  35                  40

gcc cgg ctg ggc cgc act gtg cgg ctg cag tgc cca gtg gag ggg gac      317
Ala Arg Leu Gly Arg Thr Val Arg Leu Gln Cys Pro Val Glu Gly Asp
                45                  50                  55

ccg ccg ccg ctg acc atg tgg acc aag gat ggc cgc acc atc cac agc      365
Pro Pro Pro Leu Thr Met Trp Thr Lys Asp Gly Arg Thr Ile His Ser
            60                  65                  70

ggc tgg agc cgc ttc cgc gtg ctg ccg cag ggg ctg aag gtg aag cag      413
Gly Trp Ser Arg Phe Arg Val Leu Pro Gln Gly Leu Lys Val Lys Gln
        75                  80                  85

gtg gag cgg gag gat gcc ggc gtg tac gtg tgc aag gcc acc aac ggc      461
Val Glu Arg Glu Asp Ala Gly Val Tyr Val Cys Lys Ala Thr Asn Gly
    90                  95                  100

ttc ggc agc ctg agc gtc aac tac acc ctc gtc gtg ctg gat gac att      509
Phe Gly Ser Leu Ser Val Asn Tyr Thr Leu Val Val Leu Asp Asp Ile
105                 110                 115                 120

agc cca ggg aag gag agc ctg ggg ccc gac agc tcc tct ggg ggt caa      557
Ser Pro Gly Lys Glu Ser Leu Gly Pro Asp Ser Ser Ser Gly Gly Gln
                125                 130                 135

gag gac ccc gcc agc cag cag tgg gca cga ccg cgc ttc aca cag ccc 605
Glu Asp Pro Ala Ser Gln Gln Trp Ala Arg Pro Arg Phe Thr Gln Pro
            140                 145                 150

tcc aag atg agg cgc cgg gtg atc gca cgg ccc gtg ggt agc tcc gtg 653
Ser Lys Met Arg Arg Arg Val Ile Ala Arg Pro Val Gly Ser Ser Val
        155                 160                 165

cgg ctc aag tgc gtg gcc agc ggg cac cct cgg ccc gac atc acg tgg 701
Arg Leu Lys Cys Val Ala Ser Gly His Pro Arg Pro Asp Ile Thr Trp
    170                 175                 180

atg aag gac gac cag gcc ttg acg cgc cca gag gcc gct gag ccc agg 749
Met Lys Asp Asp Gln Ala Leu Thr Arg Pro Glu Ala Ala Glu Pro Arg
185                 190                 195                 200

aag aag aag tgg aca ctg agc ctg aag aac ctg cgg ccg gag gac agc 797
Lys Lys Lys Trp Thr Leu Ser Leu Lys Asn Leu Arg Pro Glu Asp Ser
                205                 210                 215

ggc aaa tac acc tgc cgc gtg tcg aac cgc gcg ggc gcc atc aac gcc 845
Gly Lys Tyr Thr Cys Arg Val Ser Asn Arg Ala Gly Ala Ile Asn Ala
            220                 225                 230

acc tac aag gtg gat gtg atc cag cgg acc cgt tcc aag ccc gtg ctc 893
Thr Tyr Lys Val Asp Val Ile Gln Arg Thr Arg Ser Lys Pro Val Leu
        235                 240                 245

aca ggc acg cac ccc gtg aac acg acg gtg gac ttc ggg ggg acc acg 941
Thr Gly Thr His Pro Val Asn Thr Thr Val Asp Phe Gly Gly Thr Thr
    250                 255                 260

tcc ttc cag tgc aag gtg cgc agc gac gtg aag ccg gtg atc cag tgg 989
Ser Phe Gln Cys Lys Val Arg Ser Asp Val Lys Pro Val Ile Gln Trp
265                 270                 275                 280

ctg aag cgc gtg gag tac ggc gcc gag ggc cgc cac aac tcc acc atc 1037
Leu Lys Arg Val Glu Tyr Gly Ala Glu Gly Arg His Asn Ser Thr Ile
                285                 290                 295

gat gtg ggc ggc cag aag ttt gtg gtg ctg ccc acg ggt gac gtg tgg 1085
Asp Val Gly Gly Gln Lys Phe Val Val Leu Pro Thr Gly Asp Val Trp
            300                 305                 310

tcg cgg ccc gac ggc tcc tac ctc aat aag ctg ctc atc acc cgt gcc 1133
Ser Arg Pro Asp Gly Ser Tyr Leu Asn Lys Leu Leu Ile Thr Arg Ala
        315                 320                 325

cgc cag gac gat gcg ggc atg tac atc tgc ctt ggc gcc aac acc atg 1181
Arg Gln Asp Asp Ala Gly Met Tyr Ile Cys Leu Gly Ala Asn Thr Met
    330                 335                 340

ggc tac agc ttc cgc agc gcc ttc ctc acc gtg ctg cca gac cca aaa 1229
Gly Tyr Ser Phe Arg Ser Ala Phe Leu Thr Val Leu Pro Asp Pro Lys
345                 350                 355                 360

ccg cca ggg cca cct gtg gcc tcc tcg tcc tcg gcc act agc ctg ccg 1277
Pro Pro Gly Pro Pro Val Ala Ser Ser Ser Ser Ala Thr Ser Leu Pro
                365                 370                 375

tgg ccc gtg gtc atc ggc atc cca gcc ggc gct gtc ttc atc ctg ggc 1325
Trp Pro Val Val Ile Gly Ile Pro Ala Gly Ala Val Phe Ile Leu Gly

380                 385                 390

acc ctg ctc ctg tgg ctt tgc cag gcc cag aag aag ccg tgc acc ccc     1373
Thr Leu Leu Leu Trp Leu Cys Gln Ala Gln Lys Lys Pro Cys Thr Pro
        395                 400                 405

gcg cct gcc cct ccc ctg cct ggg cac cgc ccg ccg ggg acg gcc cgc     1421
Ala Pro Ala Pro Pro Leu Pro Gly His Arg Pro Pro Gly Thr Ala Arg
    410                 415                 420

gac cgc agc gga gac aag gac ctt ccc tcg ttg gcc gcc ctc agc gct     1469
Asp Arg Ser Gly Asp Lys Asp Leu Pro Ser Leu Ala Ala Leu Ser Ala
425                 430                 435                 440

ggc cct ggt gtg ggg ctg tgt gag gag cat ggg tct ccg gca gcc ccc     1517
Gly Pro Gly Val Gly Leu Cys Glu Glu His Gly Ser Pro Ala Ala Pro
                445                 450                 455

cag cac tta ctg ggc cca ggc cca gtt gct ggc cct aag ttg tac ccc     1565
Gln His Leu Leu Gly Pro Gly Pro Val Ala Gly Pro Lys Leu Tyr Pro
            460                 465                 470

aaa ctc tac aca gac atc cac aca cac aca cac aca cac tct cac aca     1613
Lys Leu Tyr Thr Asp Ile His Thr His Thr His Thr His Ser His Thr
        475                 480                 485

cac tca cac gtg gag ggc aag gtc cac cag cac atc cac tat cag tgc     1661
His Ser His Val Glu Gly Lys Val His Gln His Ile His Tyr Gln Cys
    490                 495                 500

tag acggcaccgt atctgcagtg ggcacggggg ggccggccag acaggcagac     1714

tgggaggatg gaggacggag ctgcagacga aggcagggga cccatggcga ggaggaatgg     1774

ccagcacccc aggcagtctg tgtgtgaggc atagcccctg gacacacaca cacagacaca     1834

cacactgcct ggatgcatgt atgcacacac atgcgcgcac acgtgctccc tgaaggcaca     1894

cgtacgcaca cacgcacatg cacagatatg ccgcctgggc acacagataa gctgcccaaa     1954

tgcacgcaca cgcacagaga catgccagaa catacaagga catgctgcct gaacatacac     2014

acgcacaccc atgcgcagat gtgctgcctg gacacacaca cacacacgga tatgctgtct     2074

ggacgcacac acgtgcagat atggtatccg gacacacacg tgcacagata tgctgcctgg     2134

acacacagat aatgctgcct tgacacacac atgcacggat attgcctgga cacacacaca     2194

cacacgtgtg cacagatatg ctgtctggac acgcacacac atgcagatat gctgcctgga     2254

cacacacttc cagacacacg tgcacaggcg cagatatgct gcctggacac acgcagatat     2314

gctgtctagt cacacacaca cgcagacatg ctgtccggac acacacacgc atgcacagat     2374

atgctgtccg gacacacaca cgcacgcaga tatgctgcct ggacacacac acagataatg     2434

ctgcctcaac actcacacac gtgcagatat tgcctggaca cacacatgtg cacagatatg     2494

ctgtctggac atgcacacac gtgcagatat gctgtccgga tacacacgca cgcacacatg     2554

cagatatgct gcctgggcac acacttccgg acacacatgc acacacaggt gcagatatgc     2614

tgcctggaca cacgcagact gacgtgcttt tgggagggtg tgccgtgaag cctgcagtac     2674

gtgtgccgtg aggctcatag ttgatgaggg actttccctg ctccaccgtc actcccccaa 2734

ctctgcccgc ctctgtcccc gcctcagtcc ccgcctccat ccccgcctct gtcccctggc 2794

cttggcggct atttttgcca cctgccttgg gtgcccagga gtcccctact gctgtgggct 2854

ggggttgggg gcacagcagc cccaagcctg agaggctgga gcccatggct agtggctcat 2914

ccccactgca ttctccccct gacacagaga aggggccttg gtatttatat ttaagaaatg 2974

aagataatat taataatgat ggaaggaaga ctgggttgca gggactgtgg tctctcctgg 3034

ggcccgggac ccgcctggtc tttcagccat gctgatgacc acaccccgtc caggccagac 3094

accacccccc accccactgt cgtggtggcc ccagatctct gtaattttat gtagagtttg 3154

agctgaagcc ccgtatattt aatttatttt gttaaacatg aaagtgcatc ctttccctcc 3214

a 3215

<210> 2
<211> 504
<212> PRT
<213> Homo sapiens

<400> 2

Met Thr Pro Ser Pro Leu Leu Leu Leu Leu Leu Pro Pro Leu Leu Leu
1               5                   10                  15

Gly Ala Phe Pro Pro Ala Ala Ala Ala Arg Gly Pro Pro Lys Met Ala
            20                  25                  30

Asp Lys Val Val Pro Arg Gln Val Ala Arg Leu Gly Arg Thr Val Arg
        35                  40                  45

Leu Gln Cys Pro Val Glu Gly Asp Pro Pro Pro Leu Thr Met Trp Thr
    50                  55                  60

Lys Asp Gly Arg Thr Ile His Ser Gly Trp Ser Arg Phe Arg Val Leu
65                  70                  75                  80

Pro Gln Gly Leu Lys Val Lys Gln Val Glu Arg Glu Asp Ala Gly Val
                85                  90                  95

Tyr Val Cys Lys Ala Thr Asn Gly Phe Gly Ser Leu Ser Val Asn Tyr
            100                 105                 110

Thr Leu Val Val Leu Asp Asp Ile Ser Pro Gly Lys Glu Ser Leu Gly
        115                 120                 125

Pro Asp Ser Ser Ser Gly Gly Gln Glu Asp Pro Ala Ser Gln Gln Trp
    130                 135                 140

Ala Arg Pro Arg Phe Thr Gln Pro Ser Lys Met Arg Arg Arg Val Ile
145                 150                 155                 160

Ala Arg Pro Val Gly Ser Ser Val Arg Leu Lys Cys Val Ala Ser Gly
                165                 170                 175

His Pro Arg Pro Asp Ile Thr Trp Met Lys Asp Asp Gln Ala Leu Thr
            180                 185                 190

Arg Pro Glu Ala Ala Glu Pro Arg Lys Lys Lys Trp Thr Leu Ser Leu
        195                 200                 205

Lys Asn Leu Arg Pro Glu Asp Ser Gly Lys Tyr Thr Cys Arg Val Ser
    210                 215                 220

Asn Arg Ala Gly Ala Ile Asn Ala Thr Tyr Lys Val Asp Val Ile Gln
225                 230                 235                 240

Arg Thr Arg Ser Lys Pro Val Leu Thr Gly Thr His Pro Val Asn Thr
                245                 250                 255

Thr Val Asp Phe Gly Gly Thr Thr Ser Phe Gln Cys Lys Val Arg Ser
            260                 265                 270

Asp Val Lys Pro Val Ile Gln Trp Leu Lys Arg Val Glu Tyr Gly Ala
        275                 280                 285

Glu Gly Arg His Asn Ser Thr Ile Asp Val Gly Gly Gln Lys Phe Val
    290                 295                 300

Val Leu Pro Thr Gly Asp Val Trp Ser Arg Pro Asp Gly Ser Tyr Leu
305                 310                 315                 320

Asn Lys Leu Leu Ile Thr Arg Ala Arg Gln Asp Asp Ala Gly Met Tyr
                325                 330                 335

Ile Cys Leu Gly Ala Asn Thr Met Gly Tyr Ser Phe Arg Ser Ala Phe
            340                 345                 350

Leu Thr Val Leu Pro Asp Pro Lys Pro Pro Gly Pro Pro Val Ala Ser
        355                 360                 365

Ser Ser Ser Ala Thr Ser Leu Pro Trp Pro Val Val Ile Gly Ile Pro
    370                 375                 380

Ala Gly Ala Val Phe Ile Leu Gly Thr Leu Leu Leu Trp Leu Cys Gln

385                 390                 395                 400

Ala Gln Lys Lys Pro Cys Thr Pro Ala Pro Ala Pro Pro Leu Pro Gly
                405                 410                 415

His Arg Pro Pro Gly Thr Ala Arg Asp Arg Ser Gly Asp Lys Asp Leu
            420                 425                 430

Pro Ser Leu Ala Ala Leu Ser Ala Gly Pro Gly Val Gly Leu Cys Glu
        435                 440                 445

Glu His Gly Ser Pro Ala Ala Pro Gln His Leu Leu Gly Pro Gly Pro
    450                 455                 460

Val Ala Gly Pro Lys Leu Tyr Pro Lys Leu Tyr Thr Asp Ile His Thr
465                 470                 475                 480

His Thr His Thr His Ser His Thr His Ser His Val Glu Gly Lys Val
                485                 490                 495

His Gln His Ile His Tyr Gln Cys
            500